Europäisches Patentamt

European Patent Office

Office européen des brevets

(19)

(11) **EP 1 078 637 A2**

(12) **EUROPEAN PATENT APPLICATION**

(43) Date of publication:
   **28.02.2001 Bulletin 2001/09**

(51) Int Cl.⁷: **A61K 45/06**

(21) Application number: **00307254.3**

(22) Date of filing: **23.08.2000**

| | |
|---|---|
| (84) Designated Contracting States:<br>   **AT BE CH CY DE DK ES FI FR GB GR IE IT LI LU MC NL PT SE**<br>   Designated Extension States:<br>   **AL LT LV MK RO SI**<br><br>(30) Priority: **27.08.1999 US 151089 P**<br><br>(71) Applicant: **Pfizer Products Inc.**<br>   **Groton, Connecticut 06340 (US)**<br><br>(72) Inventors:<br>   • **Coe, Jotham Wadsworth**<br>     **Groton, Connecticut 06340 (US)** | • **Harrigan, Edmund Patrick**<br>   **Groton, Connecticut 06340 (US)**<br>• **O'Neill, Brian Thomas**<br>   **Groton, Connecticut 06340 (US)**<br>• **Sands, Steven Bradley**<br>   **Groton, Connecticut 06340 (US)**<br><br>(74) Representative:<br>   **Simpson, Alison Elizabeth Fraser et al**<br>   **Urquhart-Dykes & Lord,**<br>   **30 Welbeck Street**<br>   **London W1G 8ER (GB)** |

(54) **Composition for the treatment and prevention of nicotine addiction containing a nicotine receptor agonist and an anti-depressant or anti-anxiety drug**

(57)    Pharmaceutical compositions are disclosed for the treatment of nicotine dependence or addiction, tobacco dependence or addiction, reduction of nicotine withdrawal symptoms or aiding in the cessation or lessening of tobacco use or substance abuse. The pharmaceutical compositions are comprised of a therapeutically effective combination of a nicotine receptor partial agonist and an anti-depressant or anxiolytic agent and a pharmaceutically acceptable carrier. The method of using these compounds is also disclosed.

EP 1 078 637 A2

**Description**

Background of the Invention

The present invention relates to pharmaceutical compositions for the treatment of nicotine dependence or addiction in a mammal (e.g. human) comprising a nicotine receptor partial agonist (NRPA) and an anti-depressant or anxiolytic agent. The term NRPA refers to all chemical compounds which bind at neuronal nicotinic acetylcholine specific receptor sites in mammalian tissue and elicit a partial agonist response. A partial agonist response is defined here to mean a partial, or incomplete functional effect in a given functional assay. Additionally, a partial agonist will also exhibit some degree of antagonist activity by its ability to block the action of a full agonist (Feldman, R.S., Meyer, J.S. & Quenzer, L.F. Principles of Neuropsychopharmacology, 1997; Sinauer Assoc. Inc.). The present invention may be used to treat mammals (e.g. humans) for tobacco dependence or addiction and nicotine dependence or addiction; to palliate the effects of nicotine withdrawal and to enhance the outcomes of other smoking cessation therapies.

The invention also relates to aryl fused azapolycylic compounds that bind to neuronal nicotinic acetylcholine specific receptor sites and are useful in modulating cholinergic function and are referred to in WO 9818798-A1, WO 9935131-A1 and WO 9955680-A1. The foregoing applications are owned in common with the present application and are incorporated herein by reference in their entireties.

The NRPA compounds that bind to neuronal nicotinic receptor sites can be used in combination with an anti-depressant such as for example, a tricyclic anti-depressant (e.g. amitryptyline, imipramine), a serotonin reuptake inhibitor anti-depressant (SRI) (e.g. sertraline, paroxetine, or fluoxetine), an atypical anti-depressant (bupropion, nefazodone), or a monoamine oxidase inhibitor (e.g., phenelzine, tranylcypromine) in order to treat the depression associated with addiction such as to nicotine or tobacco, alcohol dependence, cocaine addiction or tobacco or nicotine dependence independently of other psychiatric illness. The compounds that bind to neuronal nicotinic receptor sites can be used in combination with anxiolytic agents, such as for example, a benzodiazepine (e.g. diazepam, alprazolam, chlordiazepoxide) or non-benzodiazepine anxiolytics (e.g. buspirone, hydroxyzine, doxepin) in order to treat the anxiety associated with addiction such as to nicotine or tobacco, alcohol dependence, cocaine addiction or tobacco or nicotine dependence independently of other psychiatric illness.

Tobacco dependence represents the most important preventable cause of illness and death in our society, responsible for more than 400,000 deaths each year. Half of all smokers will die of diseases directly related to tobacco use, and many smokers will suffer significant morbidity.

People smoke because of the reinforcing effects of nicotine. Nicotine is a powerful psychoactive agent that activates the same brain pathways as cocaine and other psychostimulants, producing agent-associated tolerance and withdrawal effects.

Nicotine replacement therapies (NRTs) have been used for smoking cessation. These are available in the form of gum, the transdermal patch, and nasal inhaler. The gum Nicorette® (nicotine polacrilex) delivers nicotine through buccal absorption following chewing. There are also non-nicotine pharmacologic therapies for treating nicotine addiction.

Summary of Invention

The invention provides a pharmaceutical composition for treating nicotine dependence or addiction, tobacco dependence or addiction, reducing nicotine withdrawal symptoms or aiding in the cessation or lessening of tobacco use or substance abuse. The therapeutically effective pharmaceutical combination is comprised of a nicotine receptor partial agonist and an anti-depressant or anxiolytic agent and a pharmaceutically acceptable carrier.

In a more specific embodiment of the invention, the anti-depressant is selected from a tricyclic anti-depressant, a serotonin reuptake inhibitor anti-depressant (SRI), an atypical anti-depressant or a monoamine oxidase inhibitor, their pharmaceutically active salts and their optical isomers. In another more specific embodiment of the invention, the anti-depressant is selected from amitryptyline, imipramine, sertraline, paroxetine, fluoxetine, bupropion, nefazodone, phenelzine, tranylcypromine, moclobemide, venlafaxine or a pharmaceutically acceptable salt or their optical isomers thereof. A preferred antidepressant is buproprion hydrochloride or one of its optical isomers.

In another more specific embodiment of this invention, the nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;

3-benzyl-9-bromo-1,2,3,4,5,6-hexahydro-1,5-methano-pyrido[1,2-a][1,5]diazocin-8-one;

3-benzyl-9-chloro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-ethynyl-1,2,3,4,5,6-hexahydro-1,5-methano-pyrido[1,2a][1,5]diazocin-8-one;

9-(2-propenyl)-1,2,3,4,5,6-hexahydro-1,5-methano-pyrido[1,2a][1,5]diazocin-8-one;

9-(2-propyl)-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

9-(4-fluorophenyl)-1,2,3,4,5,6-hexahydro-1,5-methano-pyrido[1,2a][1,5]diazocin-8-one;

9-(3-fluorophenyl)-1,2,3,4,5,6-hexahydro-1,5-methano-pyrido[1,2a][1,5]diazocin-8-one;

9-(3,5-difluorophenyl)-1,2,3,4,5,6-hexahydro-1,5-methano-pyrido[1,2a][1,5]diazocin-8-one;

9-(2,4-difluorophenyl)-1,2,3,4,5,6-hexahydro-1,5-methano-pyrido[1,2a][1,5]diazocin-8-one;

9-(2,5-difluorophenyl)-1,2,3,4,5,6-hexahydro-1,5-methano-pyrido[1,2a][1,5]diazocin-8-one;

6-methyl-5-oxo-6,13-diazatetracyclo[$9.3.1.0^{2,10}.0^{4,8}$]pentadeca-2(10),3,8-triene;

5-oxo-6,13-diazatetracyclo[$9.3.1.0^{2,10}.0^{4,8}$]pentadeca-2(10),3,8-triene;

6-oxo-5,7,13-triazatetracyclo[$9.3.1.0^{2,10}.0^{4,8}$]pentadeca-2(10),3,8-triene;

4,5-difluoro-10-aza-tricyclo[$6.3.1.0^{2,7}$]dodeca-2(7),3,5-triene;

5-fluoro-10-aza-tricydo[$6.3.1.0^{2,7}$]dodeca-2(7),3,5-triene-4-carbonitrile;

4-ethynyl-5-fluoro-10-aza-tricyclo[$6.3.1.0^{2,7}$]dodeca-2(7),3,5-triene;

5-ethynyl-10-aza-tricyclo[$6.3.1.0^{2,7}$]dodeca-2(7),3,5-triene-4-carbonitrile;

6-methyl-5-thia-5-dioxa-6,13-diazatetracydo[$9.3.1.0^{2,10}.0^{4,8}$]pentadeca-2(10),3,8-triene;

10-aza-tricyclo[$6.3.1.0^{2,7}$]dodeca-2(7),3,5-triene;

4-fluoro-10-aza-tricyclo[$6.3.1.0^{2,7}$]dodeca-2(7),3,5-triene;

4-methyl-10-aza-tricyclo[$6.3.1.0^{2,7}$]dodeca-2(7),3,5-triene;

4-trifluoromethyl-10-aza-tricyclo[$6.3.1.0^{2,7}$]dodeca-2(7),3,5-triene;

4-nitro-10-azatricyclo[$6.3.1.0^{2,7}$]dodeca-2(7),3,5-triene;

7-methyl-5,7,13-triazatetracyclo[$9.3.1.0^{2,10}.0^{4,8}$]pentadeca-2(10),3,5,8-tetraene;

6-methyl-5,7,13-triazatetracyclo[$9.3.1.0^{2,10}.0^{4,8}$]pentadeca-2(10),3,5,8-tetraene;

6,7-dimethyl-5,7,13-triazatetracyclo[$9.3.1.0^{2,10}.0^{4,8}$]pentadeca-2(10),3,5,8-tetraene;

6-methyl-7-phenyl-5,7,13-triazatetracyclo[$9.3.1.0^{2,10}.0^{4,8}$]pentadeca-2(10),3,5,8-tetraene;

6,7-dimethyl-5,8,14-triazatetracyclo[$10.3.1.0^{2,11}.0^{4,9}$]hexadeca-2(11),3,5,7,9-pentaene;

5,8,14-triazatetracyclo[$10.3.1.0^{2,11}.0^{4,9}$]hexadeca-2(11),3,5,7,9-pentaene;

14-methyl-5,8,14-triazatetracyclo[$10.3.1.0^{2,11}.0^{4,9}$]hexadeca-2(11),3,5,7,9-pentaene;

5-oxa-7,13-diazatetracyclo[$9.3.1.0^{2,10}.0^{4,8}$]pentadeca-2(10),3,6,8-tetraene;

6-methyl-5-oxa-7,13-diazatetracyclo[$9.3.1.0^{2,10}.0^{4,8}$]pentadeca-2(10),3,6,8-tetraene;

4-chloro-10-azatricyclo[$6.3.1.0^{2,7}$]dodeca-2(7),3,5-triene;

10-azatricyclo[$6.3.1.0^{2,7}$]dodeca-2(7),3,5-trien-4-yl cyanide;

1-(10-azatricyclo[$6.3.1.0^{2,7}$]dodeca-2(7),3,5-trien-4-yl)-1-ethanone;

10-azatricyclo[$6.3.1.0^{2,7}$]dodeca-2(7),3,5-trien-4-ol;

7-methyl-5-oxa-6,13-diazatetracyclo[$9.3.1.0^{2,10}.0^{4,8}$]pentadeca-2,4(8),6,9-tetraene;

4,5-dichloro-10-azatricyclo[$6.3.1.0^{2,7}$]dodeca-2(7),3,5-triene;

11-azatricyclo[$7.3.1.0^{2,7}$]trideca-2(7),3,5-triene-5-carbonitrile;

1-[11-azatricyclo[$7.3.1.0^{2,7}$]trideca-2(7),3,5-trien-5-yl]-1-ethanone;

1-[11-azatricyclo[$7.3.1.0^{2,7}$]trideca-2(7),3,5-trien-5-yl]-1-propanone;

4-fluoro-11-azatricyclo[$7.3.1.0^{2,7}$]trideca-2(7),3,5-triene-5-carbonitrile;

5-fluoro-11-azatricyclo[$7.3.1.0^{2,7}$]trideca-2(7),3,5-triene-4-carbonitrile;

6-methyl-7-thia-5,14-diazatetracyclo[$10.3.1.0^{2,10}.0^{4,8}$]hexadeca-2(10),3,5,8-tetraene;

6-methyl-5,7,14-triazatetracyclo[$10.3.1.0^{2,10}.0^{4,8}$]hexadeca-2(10),3,5,8-tetraene;

6,7-dimethyl-5,7,14-triazatetracyclo[$10.3.1.0^{2,10}.0^{4,8}$]hexadeca-2(10),3,5,8-tetraene;

5,7,14-triazatetracyclo[$10.3.1.0^{2,10}.0^{4,8}$]hexadeca-2(10),3,5,8-tetraene;

5,6-dimethyl-5,7,14-triazatetracyclo[$10.3.1.0^{2,10}.0^{4,8}$]hexadeca-2(10),3,6,8-tetraene;

5-methyl-5,7,14-triazatetracyclo[$10.3.1.0^{2,10}.0^{4,8}$]hexadeca-2(10),3,6,8-tetraene;

6-(trifluoromethyl)-7-thia-5,14-diazatetracyclo[$10.3.1.0^{2,10}.0^{4,8}$]hexadeca-2(10),3,5,8-tetraene;

5,8,15-triazatetracyclo[$11.3.1.0^{2,11}.0^{4,9}$]heptadeca-2(11),3,5,7,9-pentaene;

7-methyl-5,8,15-triazatetracyclo[11.3.1.0$^{2,11}$.0$^{4,9}$] heptadeca-2(11),3,5,7,9-pentaene;

6-methyl-5,8,15-triazatetracyclo[11.3.1.0$^{2,11}$.0$^{4,9}$] heptadeca-2(11),3,5,7,9-pentaene;

6,7-dimethyl-5,8,15-triazatetracyclo[11.3.1.0$^{2,11}$. 0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$. 0$^{4,8}$]hexadeca-2(10),3,5,8-tetraene;

5-methyl-7-oxa-6,14-diazatetracyclo[10.3.1.0$^{2,10}$. 0$^{4,8}$]hexadeca-2(10),3,5,8-tetraene;

6-methyl-5-oxa-7,14-diazatetracyclo[10.3.1.0$^{2,10}$. 0$^{4,8}$]hexadeca-2(10),3,6,8-tetraene;

7-methyl-5-oxa-6,14-diazatetracyclo[10.3.1.0$^{2,10}$. 0$^{4,8}$]hexadeca-2(10),3,6,8-tetraene;

4,5-difluoro-11-azatricyclo[7.3.1.0$^{2,7}$]trideca-2(7), 3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7), 3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7), 3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7), 3,5-triene;

5-nitro-11-aza-tricyclo[7.3.1.0$^{2,7}$]trideca-2(7), 3,5-triene;

5-fluoro-11-aza-tricyclo[7.3.1.0$^{2,7}$]trideca-2(7), 3,5-triene; and

6-hydroxy-5-methoxy-11-aza-tricyclo[7.3.1.0$^{2,7}$]trideca-2(7),3,5-triene and

their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydrol 1,5-methano-pyrido [1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo [9.3.1.0$^{2,10}$.0$^{4,8}$]pentadeca-2(10),3,8-triene;

4-fluoro-10-aza-tricyclo[6.3.1.0$^{2,7}$]dodeca-2(7), 3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7), 3,5-triene;

6-methyl-5,7,13-triazatetracyclo[9.3.1.0$^{2,10}$.0$^{4,8}$] pentadeca-2(10),3,5,8-tetraene;

6,7-dimethyl-5,8,14-triazatetracyclo[10.3.1.0$^{2,11}$. 0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$. 0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7), 3,5-triene-5-carbonitrile;

5-fluoro-11-azatricyclo[7.3.1.0$^{2,7}$]trideca-2(7), 3,5-triene-4-carbonitrile;

6-methyl-7-thia-5,14-diazatetracyclo[10.3.1.0$^{2,10}$. 0$^{4,8}$]hexadeca-2(10),3,5,8-tetraene;

6-methyl-5,7,14-triazatetracyclo[10.3.1.0$^{2,10}$.0$^{4,8}$] hexadeca-2(10),3,5,8-tetraene;

6,7-dimethyl-5,7,14-triazatetracyclo[10.3.1.0$^{2,10}$. 0$^{4,8}$]hexadeca-2(10),3,5,8-tetraene;

6-methyl-7-oxa-5,14-diazatetracyclo[10.3.1.0$^{2,10}$. 0$^{4,8}$]hexadeca-2(10),3,5,8-tetraene;

6-methyl-5-oxa-7,14-diazatetracyclo[10.3.1.0$^{2,10}$.

$0^{4,8}$]hexadeca-2(10),3,6,8-tetraene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene; and
11-aza-tricyclo[7.3.1.0$^{2,7}$]trideca-2(7),3,5-trien-5-ol and

their pharmaceutically acceptable salts and their optical isomers.

The invention also provides a method of treating a mammal having a condition which presents with tobacco or nicotine addiction, nicotine withdrawal symptoms, alcohol dependence or cocaine or other substance addiction. The mammal is administered a nicotine receptor partial agonist or a pharmaceutically acceptable salt thereof, and an antidepressant or anxiolytic agent or a pharmaceutically acceptable salt thereof. The nicotine receptor partial agonist and the anti-depressant or anxiolytic agent are present in amounts that render the composition effective in the treatment of tobacco or nicotine addiction, nicotine withdrawal symptoms, alcohol dependence or cocaine or other substance addiction. In a more specific embodiment of the invention, the anti-depressant is selected from a tricyclic anti-depressant, a serotonin reuptake inhibitor anti-depressant, (SRI), an atypical anti-depressant, and a monoamine oxidase inhibitor. In another more specific embodiment of this invention anxiolytic agent is selected from a benzodiazepine or a non-benzodiazepine anxiolytic. In another more specific embodiment of this invention, the anxiolytic agent is a benzodiazepine or a non-benzodiazepine anxiolytic. In a more specific embodiment of the invention, the anxiolytic agent is selected from diazepam, alprazolam, chlordiazepoxide, buspirone, hydroxyzine and doxepin or a pharmaceutically acceptable salt thereof. A preferable anxiolytic is doxepin or a pharmaceutically acceptable salt or optical isomers thereof.

In another more specific embodiment of this invention the nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;

4-fluoro-10-aza-tricyclo[6.3.1.0$^{2,7}$]dodeca-2(7),3,5-triene;

4-methyl-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

7-methyl-5,7,13-triazatetracyclo[9.3.1.0$^{2,10}$.0$^{4,8}$]pentadeca-2(10),3,5,8-tetraene;

6-methyl-5,7,13-triazatetracyclo[9.3.1.0$^{2,10}$.0$^{4,8}$]pentadeca-2(10),3,5,8-tetraene;

6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3,1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene; and

6-hydroxy-5-methoxy-11-aza-tricyclo[7.3.1.0$^{2,7}$]trideca-2(7),3,5-triene and

their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo[9.3.1.0$^{2,10}$.0$^{4,8}$]pentadeca-2(10),3,8-triene;

4-fluoro-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

6-methyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

6-fluoro-11-aza-tricyclo[7.3.1.0$^{2,7}$]trideca-2(7),3,5-triene; and

11-aza-tricyclo[7,3,1.0$^{2,7}$]trideca-2(7),3,5-trien-5-ol and the pharmaceutically acceptable salts and optical isomers of the foregoing compounds.

In another more specific embodiment, the anti-depressant is selected from amitriptyline, imipramine, sertraline, paroxetine, fluoxetine, bupropion, nefazodone, phenelzine, tranylcypromine, moclobemide, venlafaxine, and the pharmaceutically acceptable salts and optical isomers isomers. A preferred anti- depressant is buprorion hydrochloride or one of its optical isomers.

The anxiolytic agent can be a benzodiazepine or a non-benzodiazepine and are selected from diazepam, alprazolam, chlordiazepoxide, buspirone, hydroxyzine or doxepin or a pharmaceutically acceptable salt or their optical isomers thereof.

A preferable anxiolytic agent is doxepin. The nicotine receptor partial agonist and the anti-depressant or anxiolytic agent can be administered substantially simultaneously.

The method also comprises administering to a mammal a nicotine receptor partial agonist or a pharmaceutically acceptable salt in amounts that render the composition effective in the treatment of tobacco or nicotine addiction, nicotine withdrawal symptoms, alcohol dependence or cocaine or other substance addiction. The nicotine partial receptor agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-ethyl-1,2,3,4,5,6-hexahydro-1,5-methano-pyrido[1,2-a][1,5]diazocin-8-one;

9-methyl-1,2,3,4,5,6-hexahydro-1,5-methano-pyrido[1,2-a][1,5]diazocin-8-one;

9-phenyl-1,2,3,4,5,6-hexahydro-1,5-methano-pyrido[1,2-a][1,5]diazocin-8-one;

9-vinyl-1,2,3,4,5,6-hexahydro-1,5-methano-pyrido[1,2-a][1,5]diazocin-8-one;

9-bromo-3-methyl-1,2,3,4,5,6-hexahydro-1,5-methano-pyrido[1,2-a][1,5]diazocin-8-one;

3-benzyl-9-bromo-1,2,3,4,5,6-hexahydro-1,5-methano-pyrido[1,2-a][1,5]diazocin-8-one;

3-benzyl-9-chloro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-ethynyl-1,2,3,4,5,6-hexahydro-1,5-methano-pyrido[1,2a][1,5]diazocin-8-one;

9-(2-propenyl)-1,2,3,4,5,6-hexahydro-1,5-methano-pyrido[1,2a][1,5]diazocin-8-one;

9-(2-propyl)-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

9-(4-fluorophenyl)-1,2,3,4,5,6-hexahydro-1,5-methano-pyrido[1,2a][1,5]diazocin-8-one;

9-(3-fluorophenyl)-1,2,3,4,5,6-hexahydro-1,5-methano-pyrido[1,2a][1,5]diazocin-8-one;

9-(3,5-difluorophenyl)-1,2,3,4,5,6-hexahydro-1,5-methano-pyrido[1,2a][1,5]diazocin-8-one;

9-(2,4-difluorophenyl)-1,2,3,4,5,6-hexahydro-1,5-methano-pyrido[1,2a][1,5]diazocin-8-one;

9-(2,5-difluorophenyl)-1,2,3,4,5,6-hexahydro-1,5-methano-pyrido[1,2a][1,5]diazocin-8-one;

6-methyl-5-oxo-6,13-diazatetracyclo[9.3.1.0$^{2,10}$.0$^{4,8}$]pentadeca-2(10),3,8-triene;

5-oxo-6,13-diazatetracyclo[9.3.1.0$^{2,10}$.0$^{4,8}$]pentadeca-2(10),3,8-triene;

6-oxo-5,7,13-triazatetracyclo[9.3.1.0$^{2,10}$.0$^{4,8}$]pentadeca-2(10),3,8-triene;

4,5-difluoro-10-aza-tricyclo[6.3.1.0$^{2,7}$]dodeca-2(7),3,5-triene;

5-fluoro-10-aza-tricyclo[6.3.1.0$^{2,7}$]dodeca-2(7),3,5-triene-4-carbonitrile;

4-ethynyl-5-fluoro-10-aza-tricyclo[6.3.1.0$^{2,7}$]dodeca-2(7),3,5-triene;

5-ethynyl-10-aza-tricyclo[6.3.1.0$^{2,7}$]dodeca-2(7),3,5-triene-4-carbonitrile;

6-methyl-5-thia-5-dioxa-6,13-diazatetracyclo[9.3.1.0$^{2,10}$.0$^{4,8}$]pentadeca-2(10),3,8-triene;

10-aza-tricyclo[6.3.1.0$^{2,7}$]dodeca-2(7),3,5-triene;

4-fluoro-10-aza-tricyclo[6.3.1.0$^{2,7}$]dodeca-2(7),3,5-triene;

4-methyl-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

7-methyl-5,7,13-triazatetracyclo[9.3.1.0$^{2,10}$.0$^{4,8}$]pentadeca-2(10),3,5,8-tetraene;

6-methyl-5,7,13-triazatetracyclo[9.3.1.0$^{2,10}$.0$^{4,8}$]pentadeca-2(10),3,5,8-tetraene;

6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$] heptadeca-2(11),3,5,7,9-pentaene;

6-methyl-5,8,15-triazatetracyclo[11.3.1.0$^{2,11}$.0$^{4,9}$] heptadeca-2(11),3,5,7,9-pentaene;

6,7-dimethyl-5,8,15-triazatetracyclo[11.3.1.0$^{2,11}$. 0$^{4,9}$]heptadeca-2(11),3,5,7,9-pentaene;

7-oxa-5,14-diazatetracydo[10.3,1,0$^{2,10}$.0$^{4,8}$]hexadeca-2(10),3,5,8-tetraene;

6-methyl-7-oxa-5,14-diazatetracyclo[10.3.1.0$^{2,10}$. 0$^{4,8}$]hexadeca-2(10),3,5,8-tetraene;

5-methyl-7-oxa-6,14-diazatetracyclo[10.3.1.0$^{2,10}$. 0$^{4,8}$]hexadeca-2(10),3,5,8-tetraene;

6-methyl-5-oxa-7,14-diazatetracyclo[10.3.1.0$^{2,10}$. 0$^{4,8}$]hexadeca-2(10),3,6,8-tetraene;

7-methyl-5-oxa-6,14-diazatetracyclo[10.3.1.0$^{2,10}$. 0$^{4,8}$]hexadeca-2(10),3,6,8-tetraene;

4,5-difluoro-11-azatricyclo[7.3.1.0$^{2,7}$]trideca-2(7), 3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7), 3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7), 3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7), 3,5-triene;

5-nitro-11-aza-tricyclo[7.3.1.0$^{2,7}$]trideca-2(7), 3,5-triene;

5-fluoro-11-aza-tricyclo[7.3.1.0$^{2,7}$]trideca-2(7), 3,5-triene;

6-hydroxy-5-methoxy-11-aza-tricyclo[7.3.1.0$^{2,7}$]trideca-2(7),3,5-triene and

their pharmaceutically acceptable salts and their optical isomers.

A preferable nicotine receptor partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido [1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methanopyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo [9.3.1.0$^{2,10}$.0$^{4,8}$]pentadeca-2(10),3,8-triene;

4-fluoro-10-aza-tricyclo[6.3.1.0$^{2,7}$]dodeca-2(7), 3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7), 3,5-triene;

6-methyl-5,7,13-triazatetracyclo[9.3.1.0$^{2,10}$.0$^{4,8}$] pentadeca-2(10),3,5,8-tetraene;

6,7-dimethyl-5,8,14-triazatetracyclo[10.3.1.0$^{2,11}$. 0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$. 0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7), 3,5-triene-5-carbonitrile;

5-fluoro-1-azatricyclo[7.3.1.0$^{2,7}$]trideca-2(7), 3,5-triene-4-carbonitrile;

6-methyl-7-thia-5,14-diazatetracyclo[10.3.1.0$^{2,10}$. 0$^{4,8}$]hexadeca-2(10),3,5,8-tetraene;

6-methyl-5,7,14-triazatetracyclo[10.3.1.0$^{2,10}$.0$^{4,8}$] hexadeca-2(10),3,5,8-tetraene;

6,7-dimethyl-5,7,14-triazatetracyclo[10.3.1.0$^{2,10}$. 0$^{4,8}$]hexadeca-2(10),3,5,8-tetraene;

6-methyl-7-oxa-5,14-diazatetracyclo[10.3.1.0$^{2,10}$. 0$^{4,8}$]hexadeca-2(10),3,5,8-tetraene;

6-methyl-5-oxa-7,14-diazatetracyclo[10.3.1.0$^{2,10}$.

0[4,8]hexadeca-2(10),3,6,8-tetraene;

5,6-difluoro-11-aza-tricyclo[7.3.1.0[2,7]]trideca-2,4,6-triene;

6-trifluoromethyl-11-aza-tricyclo[7.3.1.0[2,7]]trideca-2,4,6-triene;

6-methoxy-1-aza-tricyclo[7.3.1.0[2,7]]trideca-2(7),3,5-triene;

6-fluoro-11-aza-tricyclo[7.3.1.0[2,7]]trideca-2(7),3,5-triene;

11-aza-tricyclo[7.3.1.0[2,7]]trideca-2(7),3,5-trien-5-ol and

their pharmaceutically acceptable salts and their optical isomers.

The term "treating" as used herein, refers to reversing, alleviating, inhibiting or slowing the progress of, or preventing the disorder or condition to which such term applies, or one or more symptoms of such disorder or condition. The term "treatment", as used herein, refers to the act of treating, as "treating" is defined immediately above.

The chemist of ordinary skill will recognize that certain compounds of this invention will contain one or more atoms which may be in a particular stereochemical or geometric configuration, giving rise to stereoisomers and configurational isomers. All such isomers and mixture thereof are included in this invention. Hydrates of the compounds of this invention are also included.

The chemist of ordinary skill will recognize that certain combinations of heteroatom-containing substituent listed in this invention define compounds which will be less stable under physiological conditions (e.g., those containing acetal or animal linkages). According, such compounds are less preferred.

Detailed Description of the Invention

In combination with the NRPA, the invention includes an anti-depressant agent or a pharmaceutically acceptable salt of compounds such as a tricyclic anti-depressant (e.g. amitryptyline, imipramine), a serotonin reuptake inhibitor anti-depressant (SRI) (e.g. sertraline, paroxetine, or fluoxetine), an atypical antidepressant (bupropion, nefazodone) or a monoamine oxidase inhibitor (e.g., phenelzine, tranylcypromine), and compounds in U.S. Patent No. 4,536,518 and may be used in this invention.

In combination with the NRPA, the invention may include an anxiolytic agent or pharmaceutically acceptable salt of compounds such as a benzodiazepine (e.g. diazepam, alprazolam, chlordiazepoxide) or non-benzodiazepine anxiolytic (e.g. buspirone, hydroxyzine, doxepin).

The particular NRPA compounds listed above, which can be employed in the method and pharm, compositions of this invention, can be made by processes known in the chemical arts, for example by the methods described in WO 9818798 A1, WO 9935131-A1 and United States Provisional Patent Application No. 60/083,556 filed April 29, 1998. Some of the preparation methods useful for making the compounds of this invention may require protection of remote functionality (i.e., primary amine, secondary amine, carboxyl). The need for such protection will vary depending on the nature of the remote functionality and the conditions of the preparation methods. The need for such protection is readily determined by one skilled in the art, and is described in examples carefully described in the above cited applications. The starting materials and reagents for the NRPA compounds employed in this invention are also readily available or can be easily synthesized by those skilled in the art using conventional methods of organic synthesis. Some of the compounds used herein are related to, or are derived from compounds found in nature and accordingly many such compounds are commercially available or are reported in the literature or are easily prepared from other commonly available substances by methods which are reported in the literature.

Some of the NRPA compounds employed in this invention are ionizable at physiological conditions. Thus, for example some of the compounds of this invention are acidic and they form a salt with a pharmaceutically acceptable cation. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the NRPA compounds employed in this invention are basic, and they form a salt with a pharmaceutically acceptable anion. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, when the NRPA compounds employed in this invention form hydrates or solvates they are also within the scope of the invention.

Some of the compounds of this invention are chiral, and as such are subject to preparation via chiral synthetic routes, or separable by conventional resolution or chromatographic means. All optical forms of the compounds of this invention are within the scope of the invention.

The utility of the NRPA compounds employed in the present invention as medicinal agents in the treatment of nicotine dependence (such as tobacco dependence or addiction) in mammals (e.g. humans) is demonstrated by the activity of the compounds of this invention in conventional assays and, in particular the assays described below. These include neuronal nicotinic receptor binding, dopamine turnover, and animal models of depression (mouse behavioral despair) and anxiety (Vogel anti-conflict). Such assays also provide a means whereby the activities of the compounds of this invention can be compared between themselves and with the activities of other known compounds. The results of these comparisons are useful for determining dosage levels in mammals, including humans, for the treatment of such diseases.

Biological Assays

Procedures

Receptor binding assay: The effectiveness of the active compounds in suppressing nicotine binding to specific receptor sites is determined by the following procedure which is a modification of the methods of Lippiello, P. M. and Femandes, K. G. (in The Binding of L-[3H]Nicotine To A Single Class of High-Affinity Sites in Rat Brain Membranes, Molecular Pharm., 29, 448-54, (1986)) and Anderson, D. J. and Americ, S. P. (in Nicotinic Receptor Binding of 3H-Cystisine, 3H-Nicotine and 3H-Methylcarmbamylcholine In Rat Brain, European J. Pharm., 253, 261-67 (1994)). Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.* The rats were killed by decapitation. Brains were removed immediately following decapitation. Membranes were prepared from brain tissue according to the methods of Lippiello and Fernandez (Molec Pharmacol, 29, 448-454, (1986) with some modifications. Whole brains were removed, rinsed with ice-cold buffer, and homogenized at 0° in 10 volumes of buffer (w/v) using a Brinkmann Polytron™, setting 6, for 30 seconds. The buffer consisted of 50 mM Tris HCl at a pH of 7.5 at room temperature. The homogenate was sedimented by centrifugation (10 minutes; 50,000 x g; 0° to 4°C). The supernatant was poured off and the membranes were gently resuspended with the Polytron and centrifuged again (10 minutes; 50,000 x g; 0 to 4°C. After the second centrifugation, the membranes were resuspended in assay buffer at a concentration of 1.0g/ 100mL. The composition of the standard assay buffer was 50 mM Tris HCl, 120 mM NaCl, 5 mM KCl, 2 mM MgCl$_2$, 2 mM CaCl$_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50μL of vehicle, blank, or test compound solution, respectively. To each tube was added 200μL of [3H]-nicotine in assay buffer followed by 750μL of the membrane suspension. The final concentration of nicotine in each tube was 0.9 nM. The final concentration of cytisine in the blank was 1μM. The vehicle consisted of deionized water containing 30μL of 1 N acetic acid per 50 mL of water. The test compounds and cytisine were dissolved in vehicle. Assays were initiated by vortexing after addition of the membrane suspension to the tube. The samples were incubated at 0° to 4° C in an iced shaking water bath. Incubations were terminated by rapid filtration under vacuum through Whatman GF/B™ glass fiber filters using a Brandel™ multi-manifold tissue harvester. Following the initial filtration of the assay mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations: Specific binding (C) to the membrane is the difference between total binding in the samples containing vehicle only and membrane (A) and nonspecific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) - (B).

$$\% \text{ Inhibition} = (1-((E)/(C))) \text{ times } 100.$$

The compounds of the invention that were tested in the above assay exhibited IC$_{50}$ values of less than 10μM.

Dopamine Turnover: Rats were injected s.c. or p.o. (gavage) and then decapitated either 1 or 2 hours later. Nucleus accumbens was rapidly dissected (2 mm slices, 4°C, in 0.32 M sucrose), placed in 0.1 N perchloric acid, and then homogenized. After centrifugation 10uL of the supernatant was assayed by HPLC-ECD. Turnover/ utilization of dopamine (DA) was calculated as the ratio of tissue concentrations of metabolites ([DOPAC]+[HVA]) to DA and expressed as percent of control.

Mouse behavioral despair test: The ability of various agents to delay the onset of immobility was assayed in a behavioral despair test (Porsolt RD; Bertin A; Jalfre M|; 1979; Arch Int Pharmacodyn Ther; 229 (2) p327-36). Male CD-1 mice from Charles River, weighing 14-16 g on arrival and 25-35 g at the time of testing serve as subjects. Mice are housed 10/cage under standard laboratory conditions on a L:D/7a.m.:7p.m. lighting cycle of at least 7 days prior to experimentation. Food and water are available ad libitum until the time of testing. All compounds are administered in a volume of 10 ml/kg. Agent vehicles will depend on compound solubiltiy, but testing will typically be done using saline or distilled water as the injection vehicle.

Subjects are administered test compound (sc, ip, or po) at a predetermined pretreatment time. At the test time, groups of ten mice are placed individually in 1000 ml beakers filled with water to the 700 ml mark at 22-23°C. A five minute test is started after the last subject is placed in the beakers with ratings taken every thirty seconds. Ratings were either 1 for immobile swim or 0 for mobile swim. The ten ratings were then totaled for each subject and the data was anaylzyed with Kruskall-Wallis and Mann-Whitney U tests.

<u>Vogel Anticonflict assay</u>: The ability of various agents to increase punished responding was evaluated using a modification of the procedure described by Vogel, Beer and Clody (Psychopharmacologia 21(1); 1971). The test chambers consisted of clear plexiglass boxes (25 cm L x 22 cm W x 22 cm H) equipped with a stainless steel drinking tube and a floor of stainless steel bars, housed in sound-attenuating wooden cabinets. Training and testing were conducted between 900 and 1600 h. After 48 hours of water deprivation, rats (N=8/group) were placed into the test chambers for a training period, in which they were allowed to explore the chamber and drink water freely for up to three minutes. Animals that did not locate the drinking spout within 10 minutes were excluded from agent testing. Animals were then administered vehicle or agent (i.p.) and were placed back into the chambers for conflict testing after a 15 min agent pretreatment period. After every 20 unpunished licks, subsequent licking resulted in the presentation of a 0.5 mA current (0.5 sec duration) applied between the drinking tube and the grid floor. The number of shocks taken in a ten minute test period was recorded by computer and data were analyzed with ANOVA followed by Dunnett's t-tests for multiple comparisons to a single control. Animals that did not begin to drink within five minutes after placement in the chamber were eliminated from the experiment and behavioral disruption due to agent treatment was assumed to have occurred.

Administration of the compositions of this invention can be via any method which delivers a compound of this invention systemically and/or locally. These methods include oral routes and transdermal routes, etc. Generally, the compounds of this invention are administered orally, but parenteral administration may be utilized (e.g., intravenous, intramuscular, subcutaneous or intramedullary). The two different compounds of this invention can be co-administered simultaneously or sequentially in any order, or a single pharmaceutical composition comprising a NRPA as described above and an anti-depressant or anxiolytic as described above in a pharmaceutically acceptable carrier can be administered.

The amount and timing of compounds administered will, of course, be based on the judgement of the prescribing physician. Thus, because of patient to patient variability, the dosages given below are a guideline and the physician may titrate doses of the agent to achieve the activity that the physician considers appropriate for the individual patient. In considering the degree of activity desired, the physician must balance a variety of factors such as cognitive function, age of the patient, presence of preexisting disease, as well as presence of other diseases (e.g., cardiovascular). The following paragraphs provide preferred dosage ranges for the various components of this invention (based on average human weight of 70 kg).

In general, an effective dosage for the NRPA in the range of 0.01 to 200 mg/kg/day, preferably 0.05 to 10.0 mg/kg/day.

In particular, an effective dosage for sertraline, when used in the combination compositions and methods of this invention, is in the range of 0.01 to 1.0 mg/kg/day.

In particular, an effective dosage for paroxetine, when used in the combination compositions and methods of this invention, is in the range of 0.1 to 7.0 mg/kg/day.

In particular, an effective dosage for fluoxetine, when used in the combination compositions and methods of this invention, is in the range of 0.1 to 1.1 mg/kg/day.

In particular, an effective dosage for nefazodone, when used in the combination compositions and methods of this invention, is in the range of 1.4 to 8.6 mg/kg/day.

In particular, an effective dosage for amitryptyline, when used in the combination i compositions and methods of this invention, is in the range of 0.1 to 3.0 mg/kg/day.

In particular, an effective dosage for imipramine, when used in the combination compositions and methods of this invention, is in the range of 0.1 to 1.5 mg/kg/day.

In particular, an effective dosage for bupropion, when used in the combination compositions and methods of this invention, is in the range of 0.1 to 10.0 mg/kg/day.

In particular, an effective dosage for phenelzine, when used in the combination compositions and methods of this invention, is in the range of 1.0 to 4.3mg/kg/day

In particular, an effective dosage for tranylcypromine, when used in the combination compositions and methods of this invention, is in the range of 0.1 to 0.9 mg/kg/day

In particular, an effective dosage for moclobemide, when used in the combination compositions and methods of this invention, is in the range of 1.0 to 15 mg/kg/day

In particular, an effective dosage for venlafaxine, when used in the combination compositions and methods of this invention, is in the range of 0.1 to 5.0 mg/kg/day

In particular, an effective dosage for diazepam, when used in the combination compositions and methods of this invention, is in the range of 0.02 to 2 mg/kg/day.

In particular, an effective dosage for alprazolam, when used in the combination compositions and methods of this invention, is in the range of 0.003 to 0.2 mg/kg/day.

In particular, an effective dosage for chlordiazepoxide, when used in the combination compositions and methods of this invention, is in the range of 0.07 to 1.4 mg/kg/day.

In particular, an effective dosage for bupropion, when used in the combination compositions and methods of this invention, is in the range of 0.1 to 0.9 mg/kg/day.

In particular, an effective dosage for hydroxyzine, when used in the combination compositions and methods of this invention, is in the range of 0.14 to 6 mg/kg/day.

In particular, an effective dosage for doxepin, when used in the combination compositions and methods of this invention, is in the range of 0.3 to 4.3 mg/kg/day.

The compositions of the present invention are generally administered in the form of a pharmaceutical composition comprising at least one of the compounds of this invention together with a pharmaceutically acceptable vehicle or diluent. Thus, the compounds of this invention can be administered individually or together in any conventional oral, parenteral or transdermal dosage form.

For oral administration a pharmaceutical composition can take the form of solutions, suspensions, tablets, pills, capsules, powders, and the like. Tablets containing various excipient such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the dependence of the subject being treated.

## Claims

1. A pharmaceutical composition for treating nicotine dependence or addiction, tobacco dependence or addiction, reducing nicotine withdrawal symptoms or aiding in the cessation or lessening of tobacco use or substance abuse, comprising a therapeutically effective combination of a nicotine receptor partial agonist and an anti-depressant or anxiolytic agent, and a pharmaceutically acceptable carrier.

2. The pharmaceutical composition according to Claim 1, wherein said anti-depressant is selected from tricyclic anti-depressant, a serotonin reuptake inhibitor anti-depressant (SRI), an atypical anti-depressant or a monoamine oxidase inhibitor, their pharmaceutically active salts and their optical isomers.

3. The pharmaceutical composition according to Claim 2, wherein said anti-depressant is selected from amitryptyline, imipramine, sertraline, paroxetine, fluoxetine, buproprion nefazodone, tranylcypromine, moclobemide, venlafaxine, or phenelzine, their pharmaceutically active salts and their optical isomers.

4. The pharmaceutical composition according to Claim 1 wherein said anxiolytic agent is selected from a benzodiazepine or a non-benzodiazepine anxiolytic, their pharmaceutically active salts and their optical isomers.

5. The pharmaceutical composition according to Claim 4, wherein the anxiolytic agents are selected from diazepam, alprazolam, hydroxyzine or doxepin, their pharmaceutically active salts and their optical isomers.

6. The pharmaceutically composition according to Claim 1, wherein said nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracydo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;
4-methyl-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
7-methyl-5,7,13-triazatetracyclo[9.3.1.0$^{2,10}$.0$^{4,8}$]pentadeca-2(10),3,5,8-tetraene;
6-methyl-5,7,13-triazatetracyclo[9.3.1.0$^{2,10}$.0$^{4,8}$]pentadeca-2(10),3,5,8-tetraene;
6,7-dimethyl-5,7,13-triazatetracyclo[9.3.1.0$^{2,10}$.0$^{4,8}$]pentadeca-2(10),3,5,8-tetraene;
6-methyl-7-phenyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
14-methyl-5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracydo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1,0$^{2,7}$]trideca-2(7),3,5-triene;

5-chloro-4-fluoro-11-azatricyclo[7.3.1.0$^{2,7}$]trideca-2(7 ),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3,1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene;

6-hydroxy-5-methoxy-11-aza-tricyclo[7.3.1.0$^{2,7}$]trideca-2(7),3,5-triene; and

their pharmaceutically acceptable salts and their optical isomers.

7. A method of treating a mammal which presents with tobacco or nicotine addiction, nicotine withdrawal symptoms, alcohol dependence or cocaine or other substance addiction, comprising administering to said mammal:

    a. a nicotine receptor partial agonist or a pharmaceutically acceptable salt thereof; and
    b. an anti-depressant or anxiolytic agent or a pharmaceutically acceptable salt thereof, wherein the nicotine receptor partial agonist and the anti-depressant or anxiolytic agent are present in amounts that render the composition effective in the treatment of tobacco or nicotine addiction, nicotine withdrawals symptoms, alcohol dependence or cocaine or other substance addiction.

8. The method of daim 7, wherein the anti-depressant is selected form tricyclic anti-depressants, serotonin reuptake inhibitor anti-depressants, atypical anti-depressants, or monoamine oxidase inhibitors, and the pharmaceutically active salts and optical isomers thereof.

9. The method according to claim 7 wherein the anxiolytic agent is selected from benzodiazepine and non-benzodiazepine anxiolytic agents and their pharmaceutically acceptable salts and optical isomers.

10. The method according to claim 8 wherein the anti-depressant is selected from amitriptyline, imipramine, sertraline, paroxetine, fluoxetine, bupropion, nefazodone, moclobemide, venlafaxine, phenelzine, tranylcypromine, and the pharmaceutically acceptable salts and optical isomers thereof.

11. The method according to claim 9 wherein the anxiolytic agent is selected from diazepam, chlordiazepoxide, buspirone, hydroxyzine or doxepin or a pharmaceutically acceptable salt or an optical isomer thereof.

12. The method according to claim 8, wherein the nicotine partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;

9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetraycyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;
4-methyl-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
7-methyl-5,7,13-triazatetracyclo[9.3.1.0$^{2,10}$.0$^{4,8}$]pentadeca-2(10),3,5,8-tetraene;
6-methyl-5,7,13-triazatetracyclo[9.3.1.0$^{2,10}$.0$^{4,8}$]pentadeca-2(10),3,5,8-tetraene;
6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracydo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracydo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracydo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-1-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene;

6-hydroxy-5-methoxy-11-aza-tricyclo[7.3.1.0$^{2,7}$]trideca-2(7),3,5-triene

and a pharmaceutically acceptable salt and an optical isomer thereof.

13. The method according to claim 7, wherein the nicotine receptor partial agonist and the anti-depressant or anxiolytic agent are administered substantially simultaneously.